# EUROPÄISCHE PATENTSCHRIFT

(11) **EP 2 001 456 B1**
(45) Veröffentlichungstag und Bekanntmachung des Hinweises auf die Patenterteilung: **02.12.2009**
(21) Anmeldenummer: 07727719.2
(22) Anmeldetag: 03.04.2007
(51) Int. Cl.: A61K 31/135, A61K 31/451, A61K 31/4748, A61K 31/485, A61P 25/00

(54) **VERWENDUNG VON ZUSAMMENSETZUNGEN ENTHALTEND KAPPA-OPIOIDREZEPTOR-ANTAGONISTEN ZUR BEHANDLUNG VON DISSOZIATIVEN STÖRUNGEN**
USE OF KAPPA OPIOID RECEPTOR ANTAGONIST-CONTAINING COMPOSITIONS FOR THE TREATMENT OF DISSOCIATIVE DISORDERS
UTILISATION DE COMPOSITIONS CONTENANT DES ANTAGONISTES DES RÉCEPTEURS OPIOÏDES KAPPA POUR TRAITER DES TROUBLES DISSOCIATIFS

(30) Priorität: 04.04.2006 DE 102006015733; 11.04.2006 DE 102006016991
(43) Veröffentlichungstag der Anmeldung: 17.12.2008
(73) Patentinhaber: Emodys GmbH, 8834 Schindellegi (CH)
(72) Erfinder: Hermann, Lars-Holger, Dr., 8834 Schindellegi (CH)
(74) Vertreter: Winkler, Andreas Fritz Ernst
(86) Internationale Anmeldenummer: PCT/EP2007/053248
(87) Internationale Veröffentlichungsnummer: WO 2007/115975

(56) Entgegenhaltungen:
- EP-A- 0 451 009
- WO-A-02/13759
- WO-A-02/49643
- WO-A-03/080022
- WO-A-2005/084654
- WO-A-2005/117838
- US-B1- 6 242 456
- SIMEON D: "DEPERSONALISATION DISORDER: A CONTEMPORARY OVERVIEW" CNS DRUGS, ADIS INTERNATIONAL, AUCKLAND, NZ, Bd. 18, Nr. 6, 2004, Seiten 343-354, XP009085195 ISSN: 1172-7047
- MAGUE S D ET AL: "Antidepressant-like effects of [kappa]-opioid receptor antagonists in the forced swim test in rats" JOURNAL OF PHARMACOLOGY AND EXPERIMENTAL THERAPEUTICS 01 APR 2003 UNITED STATES, Bd. 305, Nr. 1, 1. April 2003 (2003-04-01), Seiten 323-330, XP002437983 ISSN: 0022-3565
- VADIVELU N ET AL: "Buprenorphine pharmacology and clinical applications" SEMINARS IN ANESTHESIA, SAUNDERS, CO, NEW YORK, NY, US, Bd. 23, Nr. 4, Dezember 2004 (2004-12), Seiten 281-290, XP004727515 ISSN: 0277-0326
- MAREMMANI I ET AL: "Buprenorphine as a psychoactive drug" ITALIAN JOURNAL OF PSYCHOPATHOLOGY 2006 ITALY, Bd. 12, Nr. 3, 2006, Seiten 332-341, XP002437984 ISSN: 1592-1107
- METCALF M D ET AL: "Kappa opioid antagonists: Past successes and future prospects" AAPS PHARMSCI 27 OCT 2005 UNITED STATES, Bd. 7, Nr. 3, 27. Oktober 2005 (2005-10-27), Seite 28p, XP002437985 ISSN: 1522-1059 in der Anmeldung erwähnt

## Beschreibung

### Gebiet der Erfindung

Die vorliegende Erfindung betrifft die Verwendung einer Zusammensetzung enthaltend einen *kappa-*Opioidrezeptor-Antagonisten bekannt als Buprenorphin zur Herstellung eines Medikaments zur Behandlung von emotional instabilen Persönlichkeitsstörungen (F60.3 der ICD-10) beim Menschen.

### Dissoziative Störungen

Unter dem verwendeten Oberbegriff "dissoziative Störungen" sind insbesondere spezifische Persönlichkeitsstörungen (F60 der ICD-10), emotional instabile Persönlichkeitsstörungen (F60.3 der ICD-10), kombinierte und andere Persönlichkeitsstörungen (F61 der ICD-10) sowie psychische Erkrankungen, in denen dissoziative Symptomkomplexe (gegebenenfalls neben anderen Symptomkomplexen) eine zentrale Rolle spielen, zu verstehen.

Unter dissoziativen Störungen versteht man allgemein den teilweisen oder völligen Verlust der normalen Integration der Erinnerung an die Vergangenheit, des Identitätsbewusstseins, der Wahrnehmung unmittelbarer Empfindungen sowie der Kontrolle von Körperbewegungen und Symptome, bei denen es zur Abspaltung von psychischen Funktionen wie des Erinnerungsvermögens, eigener Gefühle (beispielsweise Schmerz, Angst, Hunger, Durst), der Wahrnehmung der eigenen Person und/oder der Umgebung kommt.

Eher chronische Störungen, besonders Lähmungen und Gefühlsstörungen, entwickeln sich, wenn der Beginn der dissoziativen Störung mit unlösbaren Problemen oder interpersonalen Schwierigkeiten verbunden war. Diese Störungen wurden früher als verschiedene Formen der "Konversionsneurose oder Hysterie" klassifiziert. Sie werden als ursächlich psychogen angesehen, in enger zeitlicher Verbindung mit traumatisierenden Ereignissen, unlösbaren oder unerträglichen Konflikten oder gestörten Beziehungen. Anpassungsstörungen werden durch ein außergewöhnlich belastendes Lebensereignis ausgelöst, das eine akute Belastungsreaktion hervorruft, oder durch eine besondere Veränderung im Leben, die zu einer anhaltend unangenehmen Situation geführt hat.

Obwohl weniger schwere psychosoziale Belastungen ("life events") den Beginn und das Erscheinungsbild auch zahlreicher anderer Störungen auslösen und beeinflussen können, ist ihre ätiologische Bedeutung doch nicht immer ganz klar. Im Gegensatz dazu entstehen die im Rahmen der vorliegende Erfindung aufgeführten Störungen immer als direkte Folge einer akuten schweren Belastung oder eines kontinuierlichen Traumas. Das belastende Ereignis oder die andauernden, unangenehmen Umstände sind primäre und ausschlaggebende Kausalfaktoren, und die Störung wäre ohne ihre Einwirkung nicht entstanden.

Dissoziative Störungen betreffen allgemein dissoziative Störungen im Sinne der internationalen Klassifizierung der Krankheiten (ICD-10), dissoziative Störungen im Rahmen von Persönlichkeitsstörungen, dissoziative Störungen im Rahmen von abnormen Gewohnheiten und Störungen der Impulskontrolle und im Rahmen von posttraumatischen Belastungsstörungen. Die dissoziativen Störungen sind bevorzugt ausgewählt aus dissoziativen Störungen oder Konversionsstörungen, dissoziative Amnesie, dissoziative Fugue, dissoziativer Stupor, Trance- und Besessenheitszustände, dissoziative Bewegungsstörung, dissoziative Krampfanfälle, dissoziative Sensibilitäts- und Empfindungsstörungen, nicht näher bezeichnete dissoziative Störungen und gemischte dissoziative Störungen der genannten. Bei dem Auftreten dissoziativer Zustände bei Persönlichkeitsstörungen sind diese bevorzugt ausgewählt aus spezifischen Persönlichkeitsstörungen, paranoide Persönlichkeitsstörung, schizoide Persönlichkeitsstörung, dissoziale Persönlichkeitsstörung, emotional instabile Persönlichkeitsstörung, histrionische Persönlichkeitsstörung, anankastische Persönlichkeitsstörung, ängstliche (vermeidende) Persönlichkeitsstörung, abhängige Persönlichkeitsstörung sowie sonstige Persönlichkeitsstörungen oder nicht näher bezeichnete Persönlichkeitsstörungen, weiterhin die andauernde Persönlichkeitsänderung, die nicht folge einer Schädigung oder Krankheit des Gehirns ist, die andauernde Persönlichkeitsänderung nach Extrembelastung, die andauernde Persönlichkeitsänderung nach psychischer Krankheit sowie sonstige andauernde Persönlichkeitsänderungen oder nicht näher bezeichnete Persönlichkeitsänderungen. Die Verhaltensstörungen sind bevorzugt ausgewählt aus abnormen Gewohnheiten und Störungen der Impulskontrolle wie pathologisches Spielen, pathologische Brandstiftung (Pyromanie), pathologisches Stehlen (Kleptomanie), Trichotillomanie sowie sonstige abnorme Gewohnheiten und Störungen der Impulskontrolle und nicht näher bezeichnete abnorme Gewohnheiten und Störungen der Impulskontrolle.

### Posttraumatische Belastungsstörung

Diese entsteht als eine verzögerte oder protrahierte Reaktion auf ein belastendes Ereignis oder eine Situation kürzerer oder längerer Dauer, mit außergewöhnlicher Bedrohung oder katastrophenartigem Ausmaß, die bei fast jedem eine tiefe Verzweiflung hervorrufen würde. Typische Merkmale sind das wiederholte Erleben des Traumas in sich aufdrängenden Erinnerungen (Nachhallerinnerungen, Flashbacks), Träumen oder Alpträumen, die vor dem Hintergrund eines andauernden Gefühls von Betäubtsein und emotionaler Stumpfheit auftreten. Ferner finden sich Gleichgültigkeit gegenüber anderen Menschen, Teilnahmslosigkeit der Umgebung gegenüber und Freudlosigkeit oder ein Gefühl des Unbeteiligtseins im Sinne eines Dissoziationserlebens sowie Vermeidung von Aktivitäten und Situationen, die Erinnerungen an das Trauma wachrufen könnten. Meist tritt ein Zustand von vegetativer Übererregtheit mit Vigilanzsteigerung, einer übermäßigen Schreckhaftigkeit und Schlafstörung auf. Angst und Depression sind häufig mit den genannten Symptomen und Merkmalen assoziiert und Suizidgedanken sind nicht selten.

### Emotional instabile Persönlichkeitsstörung

Beispiele für Persönlichkeitsstörungen, die zum Teil gravierende Auswirkungen für die betreffende Person haben können, sind ferner die emotional instabile Persönlichkeitsstörung (auch als Borderline-Syndrom bekannt), aber auch andere gemischte Persönlichkeitsstörungen.

Unter emotional instabiler Persönlichkeitsstörung versteht man eine Persönlichkeitsstörung mit deutlicher Tendenz, Impulse ohne Berücksichtigung von Konsequenzen auszuagieren, verbunden mit unvorhersehbarer und launenhafter Stimmung. Es besteht eine Neigung zu emotionalen Ausbrüchen und eine Unfähigkeit, impulshaftes Verhalten zu kontrollieren. Zum Erscheinungsbild gehören sehr wechselhafte Stimmungen und Affekte, ein zerrüttetes Selbstbild, sehr unterschiedlich ausgeprägte Arten von traumabedingten Dissoziationen und damit verbundene Autoaggression.

Zur Behandlung von emotional instabiler Persönlichkeitsstörung (Borderline Syndrom) sind bereits eine Vielzahl von Therapieverfahren entwickelt worden, meistens jedoch mit nur mäßigem Erfolg.

### Aufgabe und Lösung

Aufgabe der vorliegenden Erfindung ist es, alternative und gegebenenfalls verbesserte Behandlungsmethoden von emotional instabilen Persönlichkeitsstörungen zur Verfügung zu stellen.

Die Aufgabe wird durch die Verwendung einer Zusammensetzung enthaltend einen *kappa*-Opioidrezeptor-Antagonisten bekannt als Buprenorphin zur Herstellung eines Medikaments zur Behandlung von emotional instabilen Persönlichkeitsstörungen gelöst.

Der mindestens eine *kappa*-Opioidrezeptor-Antagonist kann hierbei in reiner Form, als pharmazeutisch annehmbares Salz, Ester, Ether, Tautomer und/oder Hydrat vorliegen.

Als *kappa*-Opioidrezeptor-Antagonisten können Verbindungen oder deren pharmazeutisch annehmbare Salze, Ester, Ether, Tautomere und/oder Hydrate eingesetzt werden, wie sie beispielsweise von D. Metcalf und A. Coop in The AAPS Journal 2005; 7 (3) Art. 71 (Oktober 27, 2005) Seite 704 bis 722 beschrieben werden.

Das Opioidrezeptor-System umfasst drei Typen von heterogenen, G-Protein gekoppelten Opioidrezeptoren. Den µ(mü)-, δ- und κ(*kappa*)-Rezeptor, wobei jeder dieser Rezeptoren selektive Agonisten und Antagonisten besitzt. Antagonisten (Blocker) haben eine hohe Affinität zum Rezeptor bei fehlender oder geringer intrinsischer Aktivität (Wirkung). Den Reaktionsmechanismus der Antagonisten am Rezeptor kann man in den kompetitiven und nicht kompetitiven Antagonismus unterteilen. Kompetitiv bedeutet, dass mit einem Überschuss eines Agonisten der Antagonist gegebenenfalls vollständig vom Rezeptor verdrängt werden kann. Beim nicht kompetitiven Antagonismus ist diese Verdrängungsreaktion nicht oder nur begrenzt möglich, beispielsweise durch irreversible Bindung des Antagonisten oder durch eine Reaktion des Antagonisten mit einer anderen Stelle des Rezeptors als der Bindungsstelle des Agonisten.

Der verwendete kappa-Opioidrezeptor-Antagonist hat die folgende Struktur: (2-(N-Cyclopropylmethyl-4,5alpha-epoxy-3-hydroxy-6-methoxy-6,14-endo-ethanomorphinan-7alpha-yl)-3,3-dimethyl-2-butanol (Buprenorphin)): 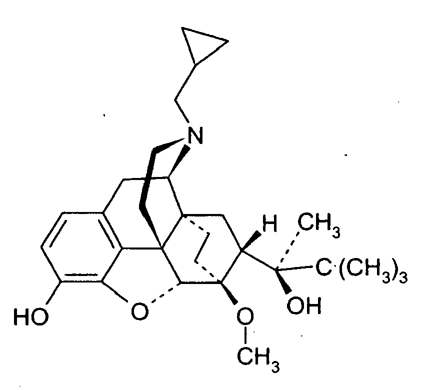 oder pharmazeutisch annehmbare Salze, Ester, Ether und/oder Hydrate davon.

Pharmazeutisch annehmbare Salze für alle im Rahmen dieser Erfindung genannten Verbindungen sind bevorzugt ausgewählt aus Chlorid, Bromid, Iodid, Sulfat, Phosphat, Tartrat, Acetat, Mucat. Die Hydrate sind bevorzugt ausgewählt aus Mono-, Di-, Tri-, Tetra- und Pentahydrat. Als Ester werden bevorzugt Carbonsäureester mit C₁₋₆-Alkyl, Acyl, Benzyl, Benzoat und dergleichen verwendet.

Gegebenfalls sind bei der Anwendung der oben genannten Verbindungen in Form von Medikamenten noch pharmazeutisch annehmbare Träger sowie Hilfs- und Zusatzstoffe beigemischt.

Pharmazeutisch annehmbare Träger sowie Hilfs- und Zusatzstoffe sind beispielsweise ein Feststoff, eine Flüssigkeit oder ein Gas. Beispiele für feste Träger sind unter anderem Lactose, Porzellanerde, Saccharose, Talk, Gelatine, Agar, Pektin, Akaziengummi, Magnesiumstearat und Stearinsäure. Beispiele für flüssige Träger sind Zuckersirup, Erdnussöl, Olivenöl und Wasser. Beispiele für gasförmige Träger sind unter anderem Kohlendioxid und Stickstoff. Beispiele für Hilfs- und Zusatzstoffe sind Verdünnungsmittel, Puffer, Granuliermittel, Gleitmittel, Sprengmittel, Bindemittel, oberflächenaktive Mittel, Verdickungsmittel sowie Farbstoffe und Pigmente, Konservierungsstoffe (einschließlich Antioxidantien), Geschmacksstoffe und Aromastoffe.

Buprenorphin, oder dessen pharmazeutisch annehmbare Salze, Ester, Ether und/oder Hydrate, werden bevorzugt in einer Menge von 1 mg bis 1000 mg eingesetzt, besonders bevorzugt von 10 mg bis 600 mg und insbesondere von 50 mg bis 500 mg, jeweils pro Darreichungsform, entsprechend 0,01 mg bis 13 mg, besonders bevorzugt 0,1 mg bis 8 mg und insbesondere 0,6 mg bis 7 mg pro Kilogramm Körpergewicht. Die Verabreichung kann in Form von Pulver, Tabletten, Lösung, Zäpfchen oder Pflaster, jeweils mit verzögerter oder ohne verzögerte Freisetzung erfolgen. Auch andere Darreichungsformen, die eine orale, intravenöse, buccale, transdermale, subkutane, rektale, inhalative, nasale oder sublinguale Verabreichung ermöglichen, sind denkbar. Bei der inhalativen Verabreichung kann es notwendig sein, weitere (Träger-)Substanzen beizumischen (Aerosole, Vernebelungshilfsstoffe). Bei der nasalen Verabreichung wird ein bereitgestelltes Pulver beispielsweise erhitzt und der entstehende Rauch eingeatmet.

In einer weiteren Ausführungsform der vorliegenden Erfindung kann Buprenorphin oder jeweils pharmazeutisch annehmbare Salze, Ester, Ether, Tautomere und/oder Hydrate davon zusammen mit mindestens einem Opioidrezeptor-Antagonisten verwendet werden.

Bevorzugte Opioidrezeptor-Antagonisten sind durch eine geringe orale Bioverfügbarkeit, bevorzugt geringer als 5%, gekennzeichnet. Derartige Substanzen unterliegen bei oraler Darreichung einem ausgeprägten First-Pass-Metabolismus und werden daher rasch abgebaut. Die im Rahmen der vorliegenden Erfindung verwendbaren Antagonisten weisen eine Bioverfügbarkeit von weniger als 5%, bevorzugt weniger als 3%, besonders bevorzugt weniger als 1 % auf. Unter Bioverfügbarkeit ist hierbei der Anteil in Prozent bzw. Gewichtsprozent des Wirkstoffes, der bei oraler Verabreichung der erfindungsgemäßen Mischung unverändert im Blut erscheint, zu verstehen. (Die Bioverfügbarkeit eines intravenös injizierten Arzneimittels ist definitionsgemäß 100%. Zur weitergehenden Definition wird auf Rainer K. Liedtke, Wörterbuch der Klinischen Pharmakologie, Gustav Fischer Verlag, Stuttgart, New York, 1980 verwiesen. Die Bioverfügbarkeit ist auch im WHO Annex 9, 1996 definiert.)

Ein besonders bevorzugter Opioidrezeptor-Antagonist ist Naloxon, das folgende Strukturformel aufweist:

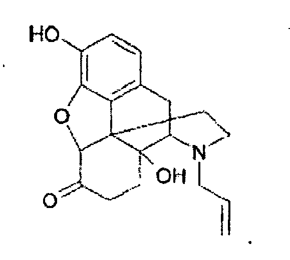

Die Opioidrezeptor-Antagonisten, insbesondere Naloxon, können gemäß der vorliegenden Erfindung in allen üblichen Darreichungsformen verabreicht werden. Bevorzugt sind physiologisch akzeptable wasserlösliche Salze wie Hydrochlorid oder Hydrochlorid-dihydrat. Der Opioidrezeptor-Antagonist kann im erfindungsgemäßen Präparat entweder in retardierter oder in nicht-retardierter Form vorliegen. Bevorzugt ist die nichtretardierte Form.

Im Rahmen der vorliegenden Erfindung denkbare und bevorzugte Kombinationen bzw. Einzelsubstanzen sind nachfolgend angegeben (*"*//*"* symbolisiert eine Kombination). Die Symbole B und C bedeuten dabei:
B = Buprenorphin oder jeweils pharmazeutisch annehmbare Salze, Ester, Ether, Tautomere und/oder Hydrate davon; C = Opioidrezeptor-Antagonist (bevorzugt Naloxon) oder pharmazeutisch annehmbare Salze, Ester, Ether, Tautomere und/oder Hydrate davon.
   - B;
   - B // C;

Im Rahmen einer besonders bevorzugten Ausführungsform der vorliegenden Erfindung wird eine Zusammensetzung, umfassend Buprenorphin, mindestens einen Opioidrezeptor-Antagonisten und gegebenenfalls mindestens einen selektiven *kappa-*Opioidrezeptor-Antagonisten verwendet.

Zur Herstellung von Buprenorphin sei auf The Merck Index, 13. Auflage, 2001, S. 252 und die darin zitierten Literaturstellen verwiesen.

Die Herstellung der erfindungsgemäßen Medikamente in Form von Pulver, Tabletten, Lösung, Zäpfchen oder Pflaster, jeweils mit verzögerter oder ohne verzögerte Freisetzung erfolgt nach im Stand der Technik bekannten Verfahren, beispielsweise durch Mischen der Komponenten, gegebenenfalls Zugabe der Träger sowie Hilfs- und Zusatzstoffe, und weiteres Mischen, gegebenenfalls Lösen oder Dispergieren der Komponenten bzw. Verpressen der Komponenten zu einer Tablette.

Gerade auch die so genannten selbstdestruktiven Verhaltensweisen mit parasuizidalen Handlungen, Selbstverletzungen und Suizidversuchen sprechen auf Buprenorphin, *kappa-*Opioidrezeptor-Antagonisten und Mischungen von Buprenorphin mit Naloxon und/oder *kappa*-Opioidrezeptor-Antagonisten an.

Die vorliegende Erfindung wird durch die nachfolgenden Herstellungsbeispiele und medizinischen Anwendungsbeispiele näher beschrieben.

### Herstellungsbeispiele:

Bei den nachfolgenden Herstellungsbeispielen werden jeweils die angegebenen arzneilich wirksamen Bestandteile mit den folgenden Hilfsstoffen vermischt und zu einer Retardfilmtablette verpresst:
Lactose-Monohydrat, Polyacrylat-Dispersion 30%, Methacrylsäure-Ethacrylat-Copolymer (1:1), Ammonium-Methacrylat-Copolymerisat Typ B, *Hypromellose 4000, Magnesiumstearat, Macrogol 6000, Talkum, Hypromellose S, Titandioxid, Eisenoxid rot E 172.

### Herstellungsbeispiel 1:

200 mg Buprenorphin.

### Herstellungsbeispiel 2:

200 mg Buprenorphin und 100 mg Binaltorphimin (BNI).

### Herstellungsbeispiel 3:

12 mg bzw. 16 mg Buprenorphin.

### Herstellungsbeispiel 4:

12 mg Buprenorphin und 2 mg Naloxon.

### Medizinische Anwendungsbeispiele:

In den medizinischen Anwendungsbeispielen 3 und 4 wird Buprenorphin alleine oder in Verbindung mit einem *kappa*-Opioidrezeptor-Antagonisten bei Patienten mit der Diagnose emotional instabiler Persönlichkeitsstörung als Möglichkeit der Behandlung der Persönlichkeitsstörung und der Reduktion der damit einhergehenden sozialen, somatischen und psychiatrischen Folgen verwendet.

### Medizinisches Anwendungsbeispiel 3:

Buprenorphin wird als mucoadhäsives Sachet mit einem Wirkstoffgehalt von 12 mg einem Patienten mit der Diagnose emotional instabiler Persönlichkeits-Störung verabreicht. Es wurde eine vollständige Remission des selbstverletzenden Verhaltens beobachtet. Die Verbesserung stabilisierte sich nach der täglichen Einnahme von 12 mg und ist auch nach einem Jahr stabil in Bezug auf das vollständige Fehlen von Selbstverletzungen. Bei zwei anderen Patientinnen fand sich eine signifikante Verringerung der Schneidehäufigkeit als zentrales Merkmal der Borderline-Erkrankung.

### Medizinisches Anwendungsbeispiel 4:

Buprenorphin wird als Sublingualtablette mit einem Wirkstoffgehalt von 12 mg und einem Naloxonanteil von 3 mg einem Patienten mit der Diagnose emotional instabile Persönlichkeitsstörung verabreicht. Hier zeigte sich eine deutliche für den Beobachtungszeitraum von zwei Jahren erkennbare Reduktion des selbstverletzenden Verhaltens. Über die klinische Wirkung hinaus konnte in den psychiatrischen Evaluationsskalen: Symptom Checklist 90 (SCL-90-R), Clinical Global Impression Scale (CGI) for severity of illness (Global Improvement and Efficacy not rated), Dissoziation Evaluation (DES), strukturiertes klinisches Interview für dissoziative Störungen SKID durchgehend eine signifikante Besserung gezeigt werden. Das Niveau der Untersuchungsskalen blieb die gesamte Untersuchungsdauer stabil.

## Patentansprüche

1. Verwendung einer Zusammensetzung, umfassend einen *kappa*-Opioidrezeptor-Antagonisten der nachfolgenden Formel zur Herstellung eines Medikaments zur Behandlung von emotional instabilen Persönlichkeitsstörungen (F60.3 der ICD-10) beim Menschen: 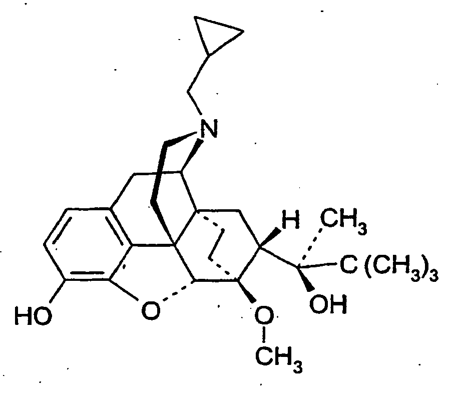 oder pharmazeutisch annehmbare Salze, Ester, Ether und/oder Hydrate davon.

2. Verwendung nach Anspruch 1, **dadurch gekennzeichnet, dass** die Zusammensetzung ferner einen Opioidrezeptor-Antagonisten umfasst.

3. Verwendung nach vorangehendem Anspruch, **dadurch gekennzeichnet, dass** der Opioidrezeptor-Antagonist Naloxon ist.

## Claims

1. Use of a composition comprising a kappa opioid receptor antagonist having the following formula for the production of a drug for the treatment of emotionally unstable personality disorders (F60.3 of ICD-10) in humans: 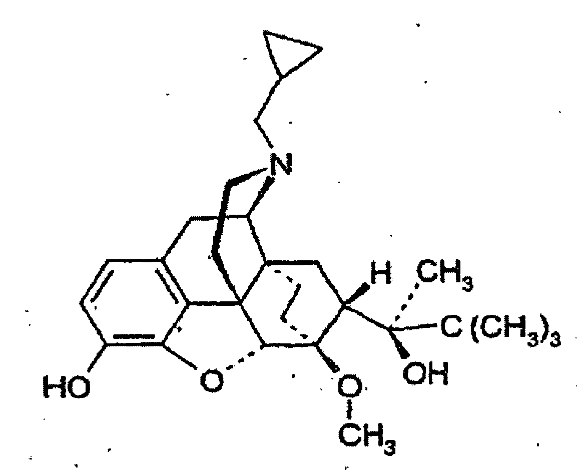 or pharmaceutically-acceptable salts, esters, ethers and/or hydrates thereof.

2. Use according to claim 1, **characterised in that** the composition also comprises an opioid receptor antagonist.

3. Use according to the preceding claim, **characterised in that** the opioid receptor antagonist is naloxone.

## Revendications

1. Utilisation d'une composition, comprenant un antagoniste de récepteurs opioïdes kappa selon la formule suivante pour produire un médicament destiné à traiter les troubles de la personnalité émotionnellement instable (F60.3 de ICD-10) chez l'être humain 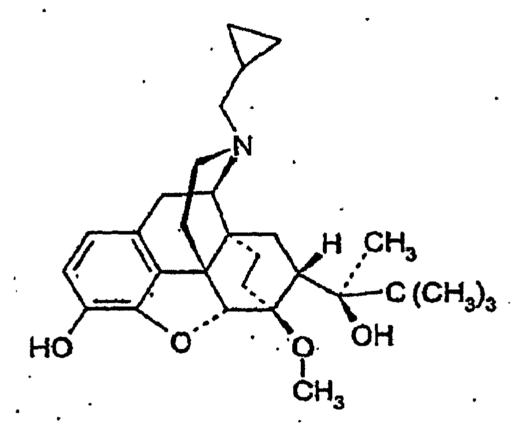 ou des sels, esters, éthers et/ou hydrates pharmaceutiquement acceptables de celui-ci.

2. Utilisation selon la revendication 1, **caractérisée en ce que** la composition comprend en outre un antagoniste des récepteurs opioïdes.

3. Utilisation selon la revendication précédente, **caractérisée en ce que** l'antagoniste des récepteurs opioïdes est la naloxone.
